# EUROPÄISCHE PATENTSCHRIFT

(11) **EP 3 161 136 B1**
(45) Veröffentlichungstag und Bekanntmachung des Hinweises auf die Patenterteilung: **20.03.2019**
(21) Anmeldenummer: 15739526.0
(22) Anmeldetag: 24.06.2015
(51) Int. Cl.: C12N 15/10

(54) **VERFAHREN ZUR ANREICHERUNG VON MIKROVESIKELN**
METHOD FOR ENRICHING MICROVESICLES
PROCÉDÉ D'ENRICHISSEMENT DE MICROVÉSICULES

(30) Priorität: 24.06.2014 DE 102014212126
(43) Veröffentlichungstag der Anmeldung: 03.05.2017
(73) Patentinhaber: AJ Innuscreen GmbH, 13125 Berlin (DE)
(72) Erfinder: HILLEBRAND, Timo, 15366 Hoppegarten (DE)
(74) Vertreter: Wehlan, Martin
(86) Internationale Anmeldenummer: PCT/EP2015/064256
(87) Internationale Veröffentlichungsnummer: WO 2015/197692

(56) Entgegenhaltungen:
- WO-A1-2009/135936
- US-A- 5 739 019
- US-A1- 2013 273 544
- ALEXANDER V VLASSOV ET AL: "Exosomes: Current knowledge of their composition, biological functions, and diagnostic and therapeutic potentials", BIOCHIMICA ET BIOPHYSICA ACTA (BBA) - GENERAL SUBJECTS, ELSEVIER, AMSTERDAM, NL, Bd. 1820, Nr. 7, 26. März 2012 (2012-03-26), Seiten 940-948, XP028509253, ISSN: 0304-4165, DOI: 10.1016/J.BBAGEN.2012.03.017 [gefunden am 2012-04-01]
- Douglas D. Taylor ET AL: "Exosome Isolation for Proteomic Analyses and RNA Profiling" In: "Clinical Applications of Mass Spectrometry", 1. Januar 2011 (2011-01-01), Humana Press, Totowa, NJ, XP055058524, ISSN: 1064-3745 ISBN: 978-1-60-761459-3 Bd. 728, Seiten 235-246, DOI: 10.1007/978-1-61779-068-3_15,

## Beschreibung

Gegenstand der Erfindung ist ein einfaches Verfahren zur Anreicherung von Mikrovesikeln (Exosomen) aus einer Probe, für eine nachfolgende Untersuchung dieser Partikel bzw. für die Freisetzung und Extraktion der in den Exosomen eingeschlossenen Nukleinsäuren, insbesondere der RNA.

Es ist bekannt, dass sich in zellfreien Körperflüssigkeiten sog. frei zirkulierende Nukleinsäuren befinden. Dabei handelt es sich um DNA als auch um RNA, welche man aus diesen Proben isolieren kann. Handelt es sich bei der sog. zellfreien zirkulierenden DNA um wirklich freie DNA, so muss davon ausgegangen werden, dass die isolierte RNA keine frei zirkulierende RNA ist, da in Körperflüssigkeiten vorkommende RNasen sehr schnell einen massiven Abbau von freier RNA bewirken würden. Es wird deshalb davon ausgegangen, dass sich die RNA in sog. Exosomen befindet.

Bei Exosomen handelt es sich um Vesikel, die von einer Zelle an ihre Umgebung abgegeben werden. Ihre Größe beträgt ca. 30 bis 90 nm.

Exosomen entstehen in einem mehrstufigen Prozess und werden final nach einem Einschnüren der Zellmembran in einem Ausschleusvorgang an die zelluläre Umgebung abgegeben. Die Exosomen enthalten Nukleinsäuren, insbesondere dabei RNA und Proteine in wechselnder Zusammensetzung. Sie dienen als Transportvehikel, zur Ausschleusung von Zellbestandteilen und nach jüngsten Kenntnissen vor allem der zellulären Kommunikation. In diesem Zusammenhang erlangen Exosomen eine immer stärkere Bedeutung für die Untersuchung verschiedener Erkrankungen (Krebs, Virusinfektionen, Autoimmunerkrankungen u.v.m.). Von besonderem Interesse ist dabei die Untersuchung von in Exosomen eingeschlossener miRNA bzw. mRNA, da diese Nukleinsäuren mittels der Exosomen aus Tumorzellen in Empfängerzellen transportiert werden und eine entscheidende Bedeutung für das Tumorwachstum zu haben scheinen. Als wichtige Ausgangsproben kommen z.B. Körperflüssigkeiten wie Blutplasma oder Serum oder auch Urin in Frage. Für andere Untersuchungsziele werden aber auch andere Probenarten eingesetzt (z.B. Milchproben). Problematisch ist, dass die Menge der in diesen Proben vorkommenden Exosomen generell sehr gering ist, so dass es wünschenswert wäre, größere Probenvolumina zu bearbeiten.

Es gibt zur Zeit nur wenige Verfahren, die es erlauben, Exosomen aus einer Probe anzureichern bzw. zu isolieren bzw. nachfolgend die in den Exosomen enthaltenen Nukleinsäuren zu extrahieren. Weit verbreitet ist die Anwendung von Ultrazentrifugationstechniken. Dabei kommt es zur Ansammlung von Exosomen am Boden des Reaktionsgefäßes. Diese Methode ist an eine Ultrazentrifuge gebunden und darüber hinaus zeitaufwendig und für die Routinediagnostik ungeeignet. Weiterhin wird auch die Technik der Ultrafiltration eingesetzt. Auch diese Methode ist zeitaufwendig und sehr teuer. Alternative Verfahren bestehen in einer Immunopräzipitation der Exosomen mittels einer Immunoplate oder mittels Immunobeads. Auch diese Art der Anreicherung der Exosomen ist zeitaufwendig und auf Grund der einzusetzenden Reagenzien störanfällig sowie teuer. Darüber hinaus können bei dieser Technologie lediglich 200 - 500 µl an Probe eingesetzt werden.

Zum Stand der Technik zählt auch die Offenlegungsschrift WO 2009/135936 A1. Gegenstand dieser Druckschrift ist ein einfaches Verfahren zur Anreicherung von Biomolekülen (z.B. Proteine oder Nukleinsäuren) aus einer Probe für die nachfolgende Isolierung der Biomoleküle und ggf. sensitive Detektion. Das Verfahren umfasst folgende Schritte:
a) Zugabe einer wässrigen Lösung eines Salzes einer Polyuronsäure zur Probe
b) Zugabe einer Substanz, welche die Gelbildung/ Pelletbildung der Polyuronsäure induziert
c) Mischen der Probe und Inkubation
d) Zentrifugation der Probe und Entfernung des Überstandes
e) Auflösen des Pellets oder Gelstückchens
f) Isolierung der Biomoleküle in an sich bekannter Art und Weise.
Als bevorzugtes Salz wird Alginat verwendet.

**Die** Isolierung und Anreicherung von Biomolekülen mittels eines Salzes einer Polyuronsäure und anschließender Gelbildung ist daher prinzipiell bekannt. Unbekannt ist hingegen, dass sich damit auch Exosomen anreichern und isolieren lassen.

**Der** Erfindung lag deshalb die Aufgabe zugrunde, eine Anreicherung von Exosomen mittels eines schnellen und einfachen Verfahrens zu ermöglichen, um insbesondere auch großvolumige Proben bearbeiten zu können. Das Verfahren soll dabei zu einer sich anschließenden einfachen und schnellen Methode zur Isolierung von RNA, die sich in den Exosomen befindet, kompatibel sein.

**Die** Aufgabe wurde gemäß den Merkmalen der Patentansprüche gelöst. Die Erfindung stellt eine Auswahlerfindung dar, nämlich die Anreicherung und Isolierung der speziellen Biomoleküle der Exosomen mittels eines Verfahrens, das für die Anreicherung und Isolierung anderer Biomoleküle bereits bekannt war. Jedoch ist diese Erfindung völlig überraschend und wird durch die Kenntnis der bekannten Druckschrift WO 2009/135936 A1 nicht nahegelegt. Obwohl es bekannt ist, Nukleinsäuren, Viren oder Proteine auf die genannte Art und Weise zu isolieren, war es völlig überraschend, dass Exosomen auch nach diesem Verfahren isolieren lassen.

Überraschenderweise wurde gefunden, dass sich bestimmte Polysaccharid-Derivate hervorragend für eine Anreicherung von Exosomen in flüssigen Proben einsetzen lassen, wobei dieser Anreicherungsschritt kompatibel zu einem effizienten Verfahren der Isolierung der in den Exosomen eingeschlossenen RNA ist. Bei den Polysaccharid- Derivaten handelt es sich um die Salze von Polyuronsäuren. Besonders geeignet sind dabei die sog. Alginate der Alginsäuren. Bei Alginaten handelt es sich um strukturgebende Elemente bei Braunalgen. Alginat ist ein Polysaccharid, welches aus 1,4 verknüpfter α-L-Guluronsäure (G) und β-D-Mannuronsäure (M) besteht.

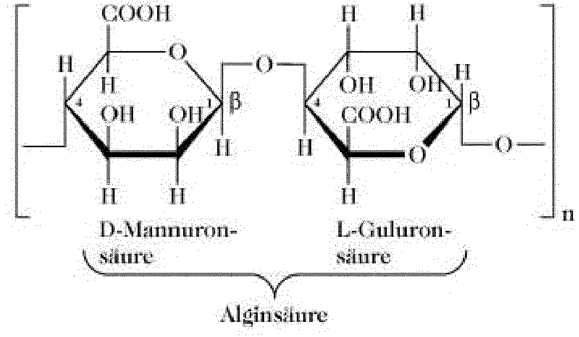

Es bildet homopolymere Bereiche, in denen Mannuronsäure oder Guluronsäure als Block vorliegt.

Alginate werden in der Lebensmittel- sowie der Pharma- und Kosmetikindustrie eingesetzt, z.B. als Geliermittel in der Lebensmittelindustrie, als Apreturmittel für Textilien, zur Herstellung von photographischen Papieren oder in der zahnmedizinischen Praxis zur Herstellung von Zahn- und Kieferabformungen.

Alginat ist auch ein wichtiges Biomaterial. Durch die Verkapselung menschlichen Zellgewebes mit Alginat ist es möglich, körperfremdes Material wie zum Beispiel Spenderzellen einzulagern, ohne dass diese vom Immunsystem erkannt und zerstört werden können. Es gibt jedoch keinerlei Hinweise darauf, das Alginate als Materialien zur Anreicherung von Exosomen für eine nachfolgende Isolierung und Aufreinigung eingeschlossener RNA eingesetzt werden können.

Für das erfindungsgemäße Verfahren nutzt man die bekannte Fähigkeit von Alginaten, in Lösungen mit niedrigem Kalziumgehalt zu gelieren und sog. Hydrogele zu bilden. Die Ursache der Gelierung begründet sich in der Einlagerung von Kalziumionen in die Zickzackstruktur der GG- Blöcke. Auf diese Zone lagert sich dann die Zickzackstruktur eines anderen Alginatmoleküls. Es kommt hierdurch zur Ausbildung dreidimensionaler Strukturen. Die Ausbildung von Gelen erfolgt auch in Kombination mit starken Säuren. Die entstandenen Gelstrukturen können darüber hinaus auch wieder spezifisch zerstört werden.

Unter Ausnutzung der Ausbildung von Alginatgelen kann die Anreicherung von Exosomen für eine nachfolgende molekulardiagnostische Analyse aus einer flüssigen Probe, insbesondere aus den problematischen großvolumigen Proben, mittels des erfindungsgemäßen Verfahrens extrem einfach und schnell realisiert werden. Das Verfahren ist dabei hinsichtlich Chemikalieneinsatz ungefährlich und benötig keinerlei Spezialausstattung.

Das erfindungsgemäße Verfahren zur Anreicherung von Exosomen aus einer Probe für eine nachfolgende Isolierung der RNA kann z.B. wie folgt ablaufen:
1. Zugabe einer wässrigen Alginatlösung zur Probe
2. Zugabe einer wässrigen Lösung, welche die Gelausbildung/ Pelletbildung induziert (z.B. Verwendung einer 1 M Kalziumchloridlösung oder einer 1%igen Salzsäurelösung)
3. Mischen der Probe und Inkubation bei Raumtemperatur
4. Zentrifugation der Probe und Entfernung des Überstandes
5. Auflösen des Pellets oder Gelstückchens und nachfolgende Isolierung der RNA in an sich bekannter Art und Weise

Die im erfindungsgemäßen Verfahren eingesetzten Konzentrationen an Alginat und Reagenz zur Gelausbildung sind extrem gering. Daraus folgt, dass der Prozess der Gelierung sichtbar gar nicht erfolgt, wie dies aus der Lebensmittelindustrie bekannt ist oder wie dies für alle Anwendung von Alginaten beschrieben ist. Nach Zentrifugation wird der Überstand entfernt. Es wird ein kleines Gelstück oder Pellet am Boden des Reaktionsgefäßes sichtbar, dieses Gelstück/ Pellet enthält die aus der Probe aufkonzentrierten Exosomen. Nachfolgend wird das Gelstück/ Pellet durch Zugabe einer Lösung, welche die Gelstruktur zerstört, wieder aufgelöst. Im letzten Schritt erfolgt dann die Isolierung der RNA aus der aufkonzentrierten Probe mit bekannten Methoden. Das Verfahren ist extrem einfach in der Durchführung. Die Inkubationszeit und nachfolgende Zentrifugation kann innerhalb von 5 - 10 min abgeschlossen sein. Die Auflösung des Gelstücks erfolgt problemlos. Es wird keine Ultrazentrifugation benötigt, so dass mit normalen Standardtischzentrifugen gearbeitet werden kann. Die Volumen der flüssigen Ausgangsproben können beliebig gewählt werden, was ein sehr breites Anwendungsspektrum erlaubt. Es kann z.B. mit Proben von 500 µl oder aber auch 10 ml gearbeitet werden. Damit ist eine enorm große Applikationsbreite gegeben.

Durch das erfindungsgemäße Verfahren reduziert sich das initiale Probenvolumen auf das im erfindungsgemäßen Prozess entstandenen Gelstück/ Pellet, welches dann problemlos im sog. Mini- Format der RNA- Isolierung weiterbearbeitet werden kann.

Für das erfindungsgemäße Verfahren kombiniert man z.B. eine wässrige Alginatlösung und eine wässrige Lösung, enthaltend Salze von di- bzw. polyvalenten Kationen (z.B. Kalziumchlorid oder Aluminiumchlorid). Ebenso kann man eine wässrige Alginatlösung und eine schwache Säure (Salzsäure) kombinieren. Zerstörung der Gelstruktur kann mittels einer Pufferlösung, welche einen Chelatbilder (EDTA) enthält oder aber auch mittels der Zugabe einer Lösung aus tri-Natriumcitrat-Dihydrat erfolgen. Ebenso kann man das Gelstück/ Pellet in einem Niedrigsalzpuffer (z.B. 10 mM Tris- HCl) bei einem basischen pH- Wert auflösen.

Eine besonders effiziente Ausführungsform nutzt den beobachteten Effekt, dass die Auflösung der entstandenen Alginatgele auch mit Puffern, welche für die Isolierung von RNA eingesetzt werden, hervorragend möglich ist. Dabei zerstören sog. chaotrope Puffer z.B. auf der Basis von Guanidiniumsalzen, Alginatgele in einer generellen Art und Weise unabhängig davon, durch welches Verfahren das Alginatgel ausgebildete wurde. Ebenso kann eine dem Fachmann bekannte Form der RNA- Isolierung unter der Verwendung einer monophasischen Lösung aus Guanidinisothyozyanat/ Phenol für eine der Anreicherung nachgeschalteten RNA Isolierung benutzt werden. Die Fachliteratur zu Alginaten und deren Anwendungsgebieten führt einen solchen beobachteten Effekt nicht auf.

Basierend auf dieser Beobachtung kann das Alginatgel dann mit einem chaotropen Puffer oder einer Mischung aus chaotroper Salzlösung und Phenol aufgelöst werden, welche nachfolgend gleichzeitig für den Prozess der Isolierung der RNA in einer Dualfunktion genutzt werden. In diesem bevorzugten Fall wird z.B. in einer Ausführungsform nach Zentrifugation und Entfernung des Überstandes das Alginatgel mit einem chaotropen Puffer aufgelöst und der Ansatz nachfolgend mit einem mineralischen Trägermaterial in Kontakt gebracht. Unter diesen Bedingungen kann die RNA an das mineralische Material binden. Optional können dem chaotropen Puffer auch noch weitere Komponenten zugegeben werden (Alkohole oder Detergenzien oder Mischungen von Alkoholen und Detergenzien), die eine Optimierung der Anbindung der RNA an das mineralische Trägermaterial verstärken können. Die gebundene RNA wird dann gewaschen und final wieder vom Trägermaterial abgelöst. Die Anreicherung von Exosomen aus einer Probe und die nachfolgende Isolierung der in den Exosomen eingeschlossenen RNA mittels dem erfindungsgemäßen Verfahrens aus einer großvolumigen Probe kann dann in der Regel schon innerhalb von ca. 30 min ermöglicht werden. Das erfindungsgemäße Verfahren ist damit deutlich einfacher und schneller als alle anderen diesbezüglichen Verfahren und auch deutlich schneller als bisher bekannte Techniken. Damit löst das erfindungsgemäße Verfahren die beschriebene Problemstellung ideal.

**Die** Erfindung wird nachfolgend anhand von Ausführungsbeispielen näher erläutert, wobei die Ausführungsbeispiele keine Limitation des erfindungsgemäßen Verfahrens bedeuten.

### Ausführungsbeispiel

### Ausführungsbeispiel : Anreicherung von Exosomen aus einer Plasmaprobe und nachfolgende Isolierung der RNA

**Die** Anreicherung und nachfolgende Isolierung der RNA erfolgte aus einer Plasmaprobe von 5 ml und wird wie folgt durchgeführt:
1. Zugabe von 100 µl einer wässrigen Alginat- Lösung (1%) sowie von 500 µl einer Kalziumchloridlösung (1M). Vortexen und bei RT für 5 min inkubieren.
2. Zentrifugation bei 4500 rpm für 10 min. Überstand vollständig entfernen.
3. Zugabe von 5 ml Wasser zum Pellet und nochmalige Zentrifugation bei 4500 rpm für 5 min.
   Nachfolgend wird die RNA mit einem kommerziellen Kit zur Isolierung von RNA (innuPREP micro RNA Kit; Analytik Jena AG) wie folgt isoliert:
4. Resuspendieren des Pellets mit 600 µl Lysis Solution RL. Zugabe von 20 µl Proteinase K und Inkubation bei RT für 15 min.
5. Überführen des Ansatzes auf eine DNA Spin Filter Säule zur Entfernung enthaltener DNA. Zentrifugation bei 12.000 rpm für 1 min. Entfernen der DNA Filter Säule
6. Zugabe eines gleichen Volumens an Isopropanol zum Filtrat, mischen der Probe und Überführen auf eine RNA Spin Filter Säule. Zentrifugation bei 12.000 rpm für 1 min. Verwerfen des Filtrates.
7. Zweimaliges Waschen der Spin Filter Säule mit ethanolhaltigen Waschpuffern (Washing Solution HS und Washing Solutiuon LS). Zentrifugation bei 12.000 rpm für 1 min. Verwerfen des Filtrates.
8. Trocknung des Spin Filter Säule durch Zentrifugation.
9. Spin Filter Säule in ein neues Reaktionsgefäß stecken. Zugabe von 50 µl RNase freiem Wasser und Elution der RNA von der Säule.

Für den Nachweis der erfolgreichen Extraktion zellfreier RNA wurde die isolierte RNA nachfolgend auf einem Agilent Bioanalyzer mit einem Small RNA Kit analysiert.

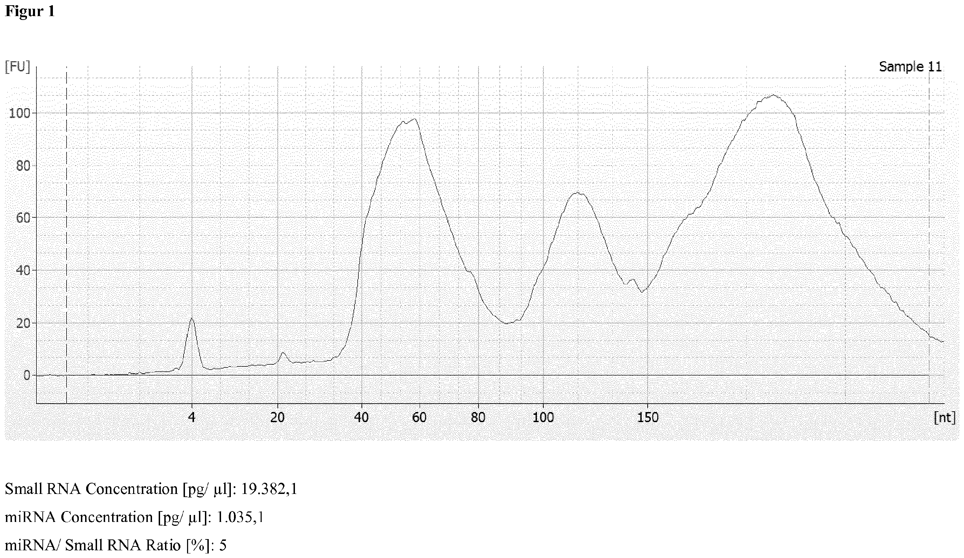

Figur 1 zeigt ein Elektropherogramm, aus dem die Anreicherung der Exosomen hervorgeht.

## Patentansprüche

1. Verfahren zur Anreicherung von Mikrovesikeln (Exosomen) aus einer Probe und nachfolgende Extraktion/ Isolierung von in diesen Mikrovesikeln enthaltenen Inhaltsstoffen, vorzugsweise RNA, **gekennzeichnet durch** folgende Schritte:
a) Zugabe einer wässrigen Lösung eines Salzes einer Polyuronsäure zur Probe
b) Zugabe einer Substanz, welche die Gelbildung/ Pelletbildung der Polyuronsäure induziert
c) Mischen der Probe und Inkubation
d) Zentrifugation der Probe und Entfernung des Überstandes
e) Auflösen des Pellets oder Gelstückchens
f) Isolierung der Inhaltsstoffe, vorzugsweise der RNA, in an sich bekannter Art und Weise.

2. Verfahren nach Anspruch 1, **dadurch gekennzeichnet, dass** es sich bei den Inhaltsstoffen um RNA oder Proteine handelt.

3. Verfahren nach Anspruch 1 oder 2, **dadurch gekennzeichnet, dass** als Salze einer Polyuronsäure Alginate eingesetzt werden.

4. Verfahren nach einem der Ansprüche 1 bis 3, **dadurch gekennzeichnet, dass** als Substanz, welche die Gelbildung/ Pelletbildung der Polyuronsäure induzieren Säuren und / oder Lösungen, die Kalziumionen enthalten, verwendet werden.

5. Verfahren nach Anspruch 4, **dadurch gekennzeichnet, dass** die Konzentration der Kalziumionen- Lösung 1 mol/ l beträgt.

6. Verfahren nach Anspruch 4, **dadurch gekennzeichnet, dass** als Säure eine 1%ige Säure verwendet wird.

7. Verfahren nach einem der Ansprüche 1 bis 6, **dadurch gekennzeichnet, dass** zum Auflösen des Pellets oder Gelstückchens Alkalien, Chelatbildner, chaotrope Salze oder Mischung chaotroper Salze mit Phenol eingesetzt werden.

8. Verfahren nach Anspruch 7, **dadurch gekennzeichnet, dass** EDTA, Guanidiniumsalze oder Citrate eingesetzt werden.

9. Verfahren nach einem der Ansprüche 1 bis 6, **dadurch gekennzeichnet, dass** vor Beginn des Verfahrens oder nach Abschluss des Verfahrens eine Lyse der Probe durchgeführt wird.

10. Verwendung von Salzen der Polyuronsäure in Kombination mit Substanzen, welche die Gelbildung/ Pelletbildung der Polyuronsäure induzieren, zur Anreicherung von Mikrovesikeln und nachfolgenden Isolierung von in den Mikrovesikeln enthaltener RNA.

## Claims

1. A method for enriching microvesicles (exosomes) from a sample and subsequent extraction / isolation of ingredients contained in said microvesicles, preferably RNA, **characterized by** the following steps:
a) Addition of an aqueous solution of a salt of a polyuronic acid to the sample
b) addition of a substance which induces gel formation / pellet formation of the polyuronic acid
c) mixing of the sample and incubation
d) centrifugation of the sample and removal of the supernatant
e) dissolving the pellet or the gel piece
f) isolation of the ingredients, preferably RNA, in manner known per se.

2. The method according to claim 1, **characterized in that** the ingredients are RNA or proteins.

3. The method according to claim 1 or 2, **characterized in that** alginates are used as salts of a polyuronic acid.

4. The method according to any one of claims 1 to 3, **characterized in that** as a substance, which induces gel formation / pellet formation of the polyuronic acid, acids and/or solutions containing calcium ions are used.

5. The method according to claim 4, **characterized in that** the concentration of the calcium ions solution is 1 mol/l.

6. The method according to claim 4, **characterized in that** a 1% acid is used as an acid.

7. The method according to any one of claims 1 to 6, **characterized in that** for dissolving the pellet or gel piece alkalis, chelating agents, chaotropic salts or a mixture of chaotropic salts with phenol are used.

8. The method according to claim 7, **characterized in that** EDTA, guanidinium salts or citrates are used.

9. The method according to any one of claims 1 to 6, **characterized in that** prior to start of 5 the process or after completion of the process a lysis of the sample is carried out.

10. A use of salts of the polyuronic acid in combination with substances, which induce gel formation / pellet formation of the polyuronic acid, for enriching microvesicles and subsequent isolation of RNA contained in the microvesicles.

## Revendications

1. Procédé d'enrichissement de microvésicules (exosomes) à partir d'un échantillon, et extraction / isolation ultérieure des ingrédients contenues dans ces microvésicules, de préférence l'ARN, **caractérisé par** les étapes suivantes:
a) addition d'une solution aqueuse d'un sel d'un acide polyuronique à l'échantillon
b) addition d'une substance induisant la gélification / la formation des pellets de l'acide polyuronique
c) mélange de l'échantillon et l'incubation
d) centrifugation de l'échantillon et l'élimination du surnageant
e) dissolution du pellet ou du morceau de gel
f) isolation des ingrédients, de préférence de l'ARN, d'une manière connue en soi.

2. Procédé selon la revendication 1, **caractérisé en ce que** les ingrédients sont l'ARN ou des protéines.

3. Procédé selon la revendication 1 ou 2, **caractérisé en ce que** des alginates sont utilisés comme sels d'un acide polyuronique.

4. Procédé selon l'une quelconque des revendications 1 à 3, **caractérisé en ce que** des acides et / ou des solutions contenant des ions calcium sont utilisés comme substance induisant la gélification / la formation des pellets de l'acide polyuronique.

5. Procédé selon la revendication 4, **caractérisé en ce que** la concentration de la solution des ions calcium est 1 mol/l.

6. Procédé selon la revendication 4, **caractérisé en ce qu'**un acide à 1% est utilisé comme acide.

7. Procédé selon l'une quelconque des revendications 1 à 6, **caractérisé en ce que** des alcalis, des agents de chélation, des sels chaotropiques ou des mélanges des sels chaotropiques avec du phénol sont utilisés pour la dissolution des pellets ou des morceaux de gel.

8. Procédé selon la revendication 7, **caractérisé en ce que** l'EDTA, des sels de guanidinium ou des citrates sont utilisés.

9. Procédé selon l'une quelconque des revendications 1 à 6, **caractérisé en ce qu'**une lyse de l'échantillon est réalisée avant le début du procédé ou après l'achèvement du procédé.

10. Utilisation des sels de l'acide polyuronique en combinaison avec des substances induisant la gélification / la formation des pellets de l'acide polyuronique pour l'enrichissement des microvésicules, et l'isolation ultérieure de l'ARN contenu dans les microvésicules.
